# EUROPEAN PATENT SPECIFICATION

(11) **EP 1 531 927 B1**
(45) Date of publication and mention of the grant of the patent: **03.12.2008**
(21) Application number: 03787967.3
(22) Date of filing: 12.08.2003
(51) Int. Cl.: B01J 13/10, A23P 1/04, A61K 8/11

(54) **PROCESS FOR THE PREPARATION OF FLAVOR OR FRAGRANCE MICROCAPSULES**
HERSTELLUNGSVERFAHREN FÜR GESCHMACK ODER DUFTMIKROKAPSELN
PROCEDE DE PREPARATION DE MICROCAPSULES AROMATIQUES OU PARFUMEES

(30) Priority: 19.08.2002 US 224441
(43) Date of publication of application: 25.05.2005
(73) Proprietor: FIRMENICH SA, 1211 Genève 8 (CH)
(72) Inventor: SUBRAMANIAM, Anandaraman, East Windsor, NJ 08520 (US); REILLY, Anne, RAHWAY NJ 07065 (US)
(74) Representative: Salvaterra-Garcia, Maria de Lurdes
(86) International application number: PCT/IB2003/003612
(87) International publication number: WO 2004/016345

(56) References cited:
- US-A- 3 803 045
- US-A- 3 956 172
- US-A- 3 965 033
- US-A- 5 023 024
- DATABASE WPI Section Ch, Week 199351 Derwent Publications Ltd., London, GB; Class D13, AN 1993-410242 XP002262916 & JP 05 309261 A (ASAMA KASEI KK), 22 November 1993 (1993-11-22)

## Description

### Technical Field

The present invention relates to the perfume and flavor industry. It relates more particularly to a coacervation process for the preparation of microcapsules encapsulating a hydrophobic flavor or fragrance ingredient or composition, the process being characterized by the use of a novel and advantageous hardening agent containing a plant extract rich in phenolic compounds.

### Background Art

Encapsulation is a term easily used by the food, flavor and fragrance industry. The processes employed for encapsulation may include dehydration techniques such as spray-drying or drum drying, spray chilling, extrusion, mechanical coating or coacervation.

Coacervation, also called aqueous phase separation, is a very well known technique for encapsulating hydrophobic liquids. A coacervation process allows to provide oil-containing microscopic capsules, the encapsulating material being a gelled hydrophilic colloid that is impervious to the oil and deposited evenly and densely about the oil as nucleus. The encapsulating colloid material is a protein which may be complexed with another colloid having an opposite electric charge.

The coacervation process essentially involves an aqueous protein solution which is manipulated by changing the physico-chemical environment (dilution and/or adjustment of pH) to result in phase separation of the protein from the solution to varying degrees depending on the molecular weight of the protein, its iso electric point and compatibility with solvents.

A coacervation process can be "simple" or "complex". The former designation is employed when a single protein is used to form a capsule wall as phase separation is taking place. The latter term designates the use of a second oppositely charged non-protein polymer to bring about the phase separation. Complex coacervation method is widely practiced in commercial processes and has been well described in the literature. In particular, US 2,800,457 and US 2,800,458 disclose complex coacervation in a very detailed manner.

Generally, a coacervation process comprises four basic steps consisting in respectively emulsification, coacervation, wall formation and wall hardening. Therefore, a dispersion of oil droplets in a solution of at least one colloid material is subjected to dilution or pH adjustment to form oil-droplet containing capsules (coacervation). The colloid is further gelled (wall formation) and is finally hardened and water-insolubilized to a point where the capsules are highly resistant to heat and moisture. More particularly, the hardening step consists in cross-linking the colloidal coating present on the emulsified oil droplets. The cross-linking of a protein, carried out in order to alter its aqueous solubility, is critical in encapsulation employing protein and is conventionally performed by the use of an aldehyde such as formaldehyde or glutaraldehyde. However, the latter compounds present the drawback of being toxic and they cannot be used in the production of microcapsules to be incorporated in foodstuffs. In particular, glutaraldehyde which is disclosed as mostly used in coacervation processes, is a hazardous chemical to handle which is moreover not permitted for applications outside the United States.

Alternatives to the use of aldehydes have been suggested in the prior art. For instance, CA 1,322,133 describes the cross-linking of gelatin by means of an irroid compound. On the other hand, J. Soper et al. in US 6,325,951 and US 6, 039,901, suggest an enzymatic cross-linking of protein-encapsulated oil particles by complex coacervation. More particularly, these patents describe a process for the preparation of a complex coacervate of oil particles, each encapsulated in a protein shell, the latter being stabilized by gelling the protein shell and subsequently enzymatically cross-linking with the help of a transglutaminase to provide thermostable microparticles. However, this enzymatic approach is expensive, hard to control from a process point of view and requires very controlled conditions to yield consistent results.

Therefore, fmding an alternative to known hardening agents, still constitutes a real need, in particular for the food and flavor industry.

Now, the present invention offers a novel solution to the above-mentioned problems by providing a coacervation process which may be of simple or complex type, and which uses as a hardening agent, i.e. as an agent capable of insolubilizing the protein wall, a plant extract presenting the particularity of being rich in phenolic compounds. This novel cross-linking agent unexpectedly proved to he very efficient and easy to handle. Moreover, it is advantageously not submitted to any kind of restriction from a legal point of view.

Phenolic compounds have been described in US 3,965,033 and US 3,803,045 as being useful in a coacervation process. However, in the processes disclosed in those documents, such compounds are added at a stage preceding the hardening step and the cross-linking is carried out with conventional hardening agents such as formaldehyde, acetaldehyde or glutaraldehyde.

Therefore, no document from the prior art has to date described a coacervation process wherein a plant extract comprising phenolic compounds is used for hardening a protein wall. The process of the present invention thus provides a novel and efficient solution to the problem of improving the cross-linking step of a coacervation process, and facilitates the preparation of microcapsules which can be advantageously used in particular in the food and flavor industry.

### Disclosure of Invention

The invention relates to a coacervation encapsulation process, comprising the basic steps of emulsification or dispersion of a hydrophobic core material in a protein solution, coacervation, wall formation by cooling the coacervate, hardening and finally stirring. This process is characterized by the fact that the hardening step is carried out by means of a plant extract rich in phenolic compounds.

The invention also relates to the microcapsules that are produced by this process. These microcapsules have gelled walls of a high molecular weight protein which at least partially surround cores of hydrophobic material, wherein the gelled walls are cross-linked by means of a hardening agent containing at least one plant extract comprising substituted or unsubstituted phenolic compounds.

The invention also relates to a method for insolubilizing proteins in water by adding at least one plant extract comprising substituted or unsubstituted phenolic compounds to a protein based coacervate wherein the extract is present in an amount sufficient to harden at least a portion of the protein of the coacervate to render it insoluble.

The invention is thus provided by a process for the preparation of microcapsules comprising a hydrophobic core material, which process comprises the steps of: a) emulsifying or dispersing a hydrophobic liquid, a solid suspended in a hydrophobic liquid or a solid into a solution of at least one high molecular weight protein ; b) subjecting the emulsion or dispersion obtained in step a) to water dilution and/or pH adjustment to achieve coacervation ; c) cooling the coacervate formed from step b) to provide wall formation of microcapsules ; d) adding at a temperature of between 15 and 30°C a hardening agent to the coacervate to cross-link the wall formed; and e) agitating the mixture for at least 48 h to finish the cross-linking reaction. This process is characterized in that the hardening agent used in step d) contains at least one plant extract comprising substituted or unsubstituted phenolic compounds.

By "high molecular weight protein" we mean here a protein with a molecular weight typically comprised between 40000 and 100000.

The terms "core material" encompass hydrophobic liquid materials which are usually subjected to encapsulation by coacervation, as well as solids or solids suspended in a hydrophobic liquid.

The plant extract used in the process according to the invention constitutes a novel agent for insolubilizing the protein wall of an encapsulated hydrophobic core material, prepared by coacervation. Cross-linking of a protein is critical for efficiency of the protein-encapsulated core, in particular for its thermostability. If a protein wall is not cross-linked, the delivery system will not perform as desired. Now, the new hardening agent used according to the invention and consisting of at least one plant extract characterized by the fact that it comprises unsubstituted or substituted phenolic compounds, unexpectedly proved to efficiently cross-link a protein wall formed after gelation. The delivery systems provided by the process here-described turned out to show a very interesting slow-release of the core material there-encapsulated, compared with traditional systems cross-linked with glutaraldehyde. Moreover, the cross-linked walls of the microcapsules present a good thermostability. On the other hand, the novel hardening agent provided by the present invention presents the advantage of being non hazardous to handle, of low cost, and it does not require rigorously controlled conditions in the process. Other advantages of the invention will appear further in the description, as well as in the examples below.

By "plant extract", it is meant here a botanical extract, an herbal extract or yet a wood extract. Non limiting examples of botanical extracts include for instance oak bark extract, olive water or even cashew nut shell liquid. Tea extract is another example of a plant extract suitable for the purpose of the invention. All these extracts comprise a certain amount of substituted or unsubstituted phenolic compounds, i.e. phenols, their homologues, or substituted phenols such as vanillin, quinones or polyphenols for instance.

In a particular embodiment of the invention, one will use as a plant extract suitable for the cross-linking of the wall protein, a liquid smoke, also referred to as pyroligneous acid. This material is originally extracted from wood, preferably birch wood. It is described by S. Arctander in Flavor and Fragrance Chemicals, Montclair, New Jersey, 1969, ref. No 2780, as being a flavor ingredient, useful in the flavoring of meats, fishes and other preserves, and to a lesser extent in flavor compositions for imitation of caramel, butterscotch, rum, vanilla, etc. in tobacco flavoring. Pyroligneous acid extract is further mentioned as being used as "smoke" flavor in products such as meat and fish. This extract comprises, in particular compounds such as guaiacol, 4-methyl guaiacol, 4-ethyl guaiacol, 4-propyl guaiacol, vanillin, 4-(2-propio) vanillone, 4-(1-propio) vanillone, aceto vanillone, eugenol, E-isoeugenol, Z-isoeugenol, syringol, 4-methyl syringol, 4-ethyl syringol, 4-propyl syringol, syringaldehyde, 4-(2-propio)-syringone, 4-(1-propio)-syringone, 4-(2-propenyl) syringol, E-4(1-propenyl) syringol, Z-4(1-propenyl) syringol and acetosyringol.

In this particular embodiment of the invention, namely wherein a "smoke" flavor is used as a hardening agent in the coacervation process, the organoleptic characteristics of the hydrophobic core encapsulated, in particular its flavor in the case of a flavor oil, are advantageously not altered. In other words, despite the fact that pyroligneous acid possesses itself flavoring properties as mentioned in reference text books such as that of S. Arctander, it turned out that the smoke flavor is not perceived in the final delivery system cross-linked with the ingredient. This advantage is quite unexpected given that liquid smoke is disclosed as being very strong and hard to mask.

In another particular embodiment of the invention, the plant extract used for the cross-linking of the protein may be used in conjunction with tannic acid.

In the process according to the invention, the hardening agent is used in proportions varying between 0.5 to 2.8% by weight relative to the weight of protein used, on a dry weight basis.

The process of the invention starts with the emulsification or dispersion of a hydrophobic liquid, a solid suspended in a hydrophobic liquid or a solid, into a protein solution.

As mentioned above, both simple and complex coacervation can be performed within the frame of the present invention. The former method involves the addition of a non-solvent or another chemical that competes for solubility with protein, resulting in a protein rich coacervate phase. On the other hand, the complex coacervation process employs the addition of a second oppositely charged polymer solution to neutralize the charges of the protein molecules, resulting in a coacervate with a neutral polymer-polymer complex. The mixture may be made by forming an aqueous solution of protein, emulsifying the core material therein, and mixing the emulsion with an aqueous solution of anionic polymer, or the two solutions may be made and mixed and the core material emulsified therein. Any anionic polymer that reacts with the protein to form complex coacervates may be employed. In particular, gum Arabic, sodium alginate, agar, carrageenan, carboxymethyl cellulose, sodium polyacrylate or polyphosphoric acid are suitable anionic polymers for the purpose of the invention. Both techniques are well known and largely described in the prior art, for instance by John C. Soper in "Utilization of Coacervated Flavors", Proceedings of Flavor Symposium; American Society, Chap. 10, 1995.

The protein is an essential element of the process according to the invention, as it will go under gelation in order to form a wall around the oil droplets, before being hardened. Use of proteins for encapsulation is limited but versatile, primarily due to their gelling and solubility characteristics. These characteristics are manipulated by changes in temperature, pH, addition of a second polyelectrolyte solution, a second solvent or even ionizing salt solution.

The molecular weight of the protein is typically of the order of 40000 to 100000. In a particular embodiment of the invention, the protein used in the process is chosen from group consisting of gelatin and albumin.

Preferably, one will use gelatin. There is no particular limitation on the gelatin that can be used but it is preferable to use gelatin having good physicochemical and chemical properties as typified by good film forming ability, the nature of an ampholyte, the controllability of the quantity of charges by pH, and the occurrence of the change from solution to gel at a critical temperature. Stated specifically, any gelatin that satisfies the specification for use in the production of microcapsules may be employed. More preferably, a gelatin having an isoelectric point of 3.5 to10 and a bloom strength of 225 to 325 is used.

In one embodiment of the invention, the core material to be encapsulated is a perfume or flavor ingredient or a perfume or flavor composition. The terms "perfume and flavor ingredient or composition " as used herein are deemed to define a variety of flavor and fragrance materials of both natural and synthetic origins. They include single compounds or mixture. The process of the invention can provide in particular delivery system for hydrophobic volatile or labile components in liquid form. Specific examples of such components may be found in the current literature, e.g. in Fenaroli's Handbook of Flavor Ingredients, 1975, CRC Press; Synthetic Food Adjuncts, 1947 by M.B. Jacobs, edited by Van Nostrand ; or Perfume and Flavor Chemicals by S. Arctander 1969, Montclair, N.J. (USA). These substances are well known to the person skilled in the art of perfuming, flavoring and/or aromatizing consumer products, i.e. of imparting an odor and/or a flavor or taste to a consumer product traditionally perfumed or flavored, or of modifying the odor and/or taste of the consumer product.

Natural extracts can also be encapsulated into the system of the invention ; these include e.g., citrus extracts such as lemon, orange, lime, grapefruit or mandarin oils, or coffee, tea, mint, cocoa, vanilla or essential oils of herbs and spices, among others.

The proportions of hydrophobic ingredient or composition are comprised between 35 and 90% by weight relative to the weight of protein used.

The process of the invention is also suitable for the encapsulation of other core materials than perfuming or flavoring ingredients. It can be used for instance to prepare delivery systems for pharmaceuticals or cosmetic ingredients.

In the second step of the process of the invention, the emulsion or dispersion obtained under step a) is subjected to water dilution and/or pH adjustment to achieve coacervation. The first two steps have to be carried out at a temperature above the gel point of the colloid material used.

The formed coacervate is further cooled to a temperature at or below the gel point of the protein to provide wall formation of microcapsules. Typically, the temperature is between 5 and 15°C depending on the gelatin source. The cooling rate is usually comprised between 0.25°/min and 1°/min. The plant extract constituting the hardening agent is then added at a temperature between 15 and 30°C and a pH preferably between 3.5 and 7, and finally gentle agitation is provided for at least 48 h. The plant extract components thus react with the protein wall and render it insoluble in water.

Other process characteristics will be specified in the examples below. Variations of the here-above described coacervation process can be considered within the framework of the invention. The description of the basic steps of the process of the invention should thus not be interpreted as limiting the invention, as a skilled person in the art knows well alternative or particular ways of carrying out the process which will not alter the invention character, as long as a hardening agent as defined above is used. For instance, an optional step of spray-drying the coacervate can be used to obtain a dry and free-flowing powder.

The delivery system obtainable by the above-described process is also a feature of the present invention. Cross-linking of the protein to alter aqueous solubility is critical in encapsulation employing proteins. The products obtained according to the present invention, due to the novel cross-linking agent, show advantageous release characteristics compared with a classical coacervate cross-linked with glutaraldehyde as shown by the comparative example below.

The microcapsules produced by the process of the invention can be used in many kinds of applications in the field of flavors and fragrances. In particular, they can be used for the flavoring of baking applications, tobacco, frying and canning (thermal processing). On the other hand, in the field of perfumery, they can be used for the perfuming of varied consumer products such as household cleaners, premoistened wipes and personal care products. Therefore perfuming or flavoring compositions comprising microcapsules according to the invention together with other perfuming or flavoring co-ingredients, are also embodiments of the present invention.

Finally, another embodiment of the invention relates to a method for insolubilizing proteins in water by adding a plant extract comprising substituted or unsubstituted phenolic compounds to a protein based coacervate.

### Brief Description of Drawings

Figure 1 (Fig. 1) represents steam distillation rates of unencapsulated orange oil and orange oil coacervate cross-linked with 0.14% aqueous glutaraldehyde solution 50% concentration.
Figure 2 (Fig. 2) represents steam distillation rate of coacervate cross-linked with 0.28% liquid smoke (Charsol^{®} Supreme ; origin: Red Arrow company LLC, Wisconsin, USA).

### Modes for Carrying out the Invention

The invention will be now described in a more detailed manner in the examples below, wherein the temperatures are indicated in degrees Celsius and the abbreviations have the usual meaning in the art.

### Example 1

### Preparation of an orange peel oil delivery system

Cold pressed orange peel oil was encapsulated by coacervation according to the formula below:

| Ingredients | Grams | % dry |
|---|---|---|
| 275 Bloom Type A Gelatin (8.25% solution) | 109 | 20.70 |
| Gum Arabic (3.85% solution) | 156 | 29.63 |
| Orange peel oil | 60 | 11.40 |
| Demineralized water | 200 | 37.99 |
| Liquid smoke ¹⁾ | 1.47 | 0.28 |
| Total | | 100.00 |

| | | |
|---|---|---|
| 1) 2 kinds of samples were used : a) Charsol^{®} Supreme (water base, 15-23 mg/ml smoke components); b) Charoil^{®} Hickory (soybean oil base, 7.3-8.1 mg/ml smoke compounds) ; origin : Red Arrow Company LLC, Wisconsin, USA | | |

### Procedure for preparation :

The solutions of gelatin on the one hand and gum Arabic on the other were each heated in a waterbath at 60°C. Upon complete dissolution, the solutions were cooled to 50°C and mixed together with agitation using magnetic stirring. The orange peel oil was added and the agitation continued for about 1 min. Water dilution (45°C) was added and agitation continued. The contents of the beaker were allowed to cool to 15°C with continued agitation. Through out the cooling period, the progress of coacervation and coating of particulate was monitored by microscopy. At the end, when no free coacervated protein was observable, liquid smoke was added and the mixture was kept under agitation for 48 h (minimum).

Samples were stored at 40°C for 24 h and subsequently examined under microscope. These samples were tested for wall integrity by heating to 90°C for 15 min and examined under microscope.

Additional samples were prepared using Charsol^{®} Supreme (water base) at 0.7% and 0.14% levels in the formulation. These samples were tested similarly.

The test results are summarized in Table 1.

**Table 1: Coacervate stored at 40° for 24 h and heated at 90° for 15 min.**

| Liquid smoke type | Level [%] | Observations | |
|---|---|---|---|
| | | 90°C - 15 min | 40°C - 24h |
| Charsol^{®} Supreme | 0.28 | Intact walls - Discrete particles | Intact walls - Discrete particles |
| Charsol^{®} Supreme | 0.14 | Intact walls - Walls fused | Intact walls - Walls fused |
| Charsol^{®} Supreme | 0.07 | Intact walls - Discrete particles | Intact walls - Discrete particles |
| Charoil^{®} Hichory | 0.28 | Intact walls - Discrete particles | Intact walls - Discrete particles |

### Example 2

### Steam distillation of delivery systems prepared according to Example 1 - Comparison between liquid smoke and glutaraldehyde

Steam distillation was done on the sample prepared according to Example 1 containing 0.28% Charsol^{®} Supreme, to verify extent of cross-linking and release rate. The same experiment was done with a coacervate cross-linked with 0.14% aqueous glutaraldehyde (solution 50% concentration).

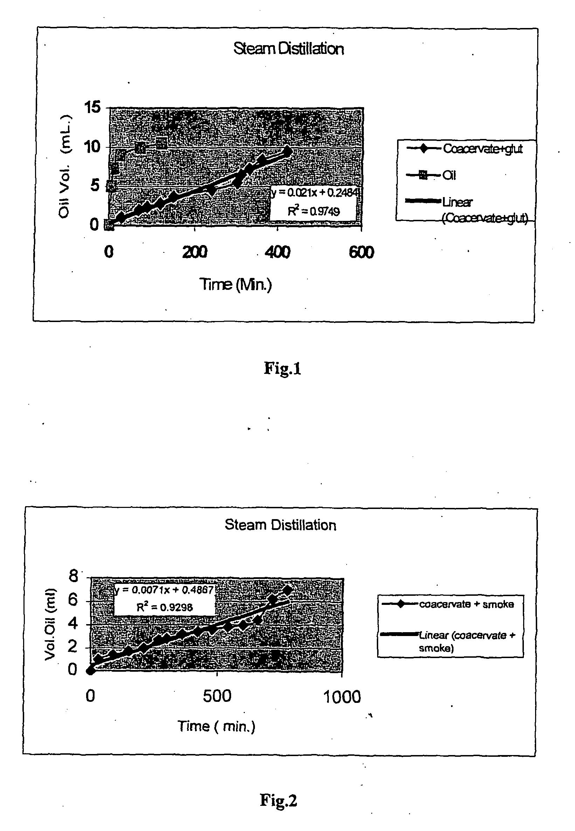

Steam distillation rates for each sample are represented in Fig. 1 and Fig. 2. It can be established that the release rate from coacervate cross-linked with 0.28% aqueous liquid smoke (Charcol^{®} Supreme) is, advantageously, only one third of a sample cross-linked with 0.14% aqueous glutaraldehyde, as evidenced by the respective slope of the regression curves.

## Claims

1. A process for the preparation of microcapsules comprising a hydrophobic core material, which process comprises the steps of :
a) emulsifying or dispersing a hydrophobic liquid, a solid suspended in a hydrophobic liquid or a solid into a solution of at least one high molecular weight protein;
b) subjecting the emulsion or dispersion obtained in step a) to water dilution and/or pH adjustment to achieve coacervation ;
c) cooling the coacervate formed from step b) to provide wall formation of microcapsules ;
d) adding at a temperature of between 15 and 30°C a hardening agent to the coacervate to cross-link the wall formed; and
e) agitating the mixture for at least 48 h to finish the cross-linking reaction;
**characterized in that** the hardening agent used in step d) contains at least one plant extract comprising substituted or unsubstituted phenolic compounds.

2. The process according to claim 1, **characterized in that** the hardening agent represents from 0.5 to 2.5% by weight relative to the weight of the protein, on a dry basis.

3. The process according to claim 1, **characterized in that** the hardening agent contains at least one plant extract selected from the group consisting of botanical extracts, herbal extracts and wood extracts.

4. The process according to claim 3, **characterized in that** the hardening agent contains a concentrated tea extract.

5. The process according to claim 3, **characterized in that** the hardening agent contains pyroligneous acid.

6. The process according to claim 1, **characterized in that** the hydrophobic core material is a perfuming or flavoring ingredient or composition.

7. The process according to claim 1, **characterized in that** the protein solution further comprises an anionic polymer.

8. The process according to claim 7, **characterized in that** the anionic polymer is selected from the group consisting of gum Arabic, sodium alginate, agar, carrageenan, carboxymethyl cellulose, sodium polyacrylate or polyphosphoric acid.

9. The process according to claim 1, **characterized in that** the protein consists of gelatin.

10. Microcapsules obtained by a process according to claim 1.

11. Microcapsules comprising gelled walls of a high molecular weight protein which at least partially surround cores of hydrophobic material, wherein the gelled walls are cross-linked by means of a hardening agent containing at least one plant extract comprising substituted or unsubstituted phenolic compounds.

12. The microcapsules of claim 11 **characterized in that** the protein is gelatin or albumin and the plant extract is selected from the group consisting of botanical extracts, herbal extracts and wood extracts.

13. A method for insolubilizing proteins in water, which comprises adding at least one plant extract comprising substituted or unsubstituted phenolic compounds to a protein based coacervate to form a mixture wherein the extract is present in an amount sufficient to harden at least a portion of the protein of the coacervate to render it insoluble.

14. A method for imparting, improving or modifying the organoleptic properties of a perfuming or flavoring composition, which comprises adding to the composition microcapsules according to claim 10.

15. A perfuming composition or a perfumed article comprising, together with perfuming co-ingredients, solvents or adjuvants or current use in perfumery, microcapsules according to claim 10.

16. A perfumed article according to claim 15, in the form of a household cleaner, a premoistened wipe or a personal care product.

17. A flavoring composition or a flavored product comprising, together with flavoring co-ingredients, or adjuvants of current use in the flavor industry, microcapsules according to claim 10.

18. A flavored product according to claim 17, in the form of a baked product, tobacco, frying or canning.

## Patentansprüche

1. Verfahren zur Herstellung von Mikrokapseln umfassend ein hydrophobes Kernmaterial, wobei das Verfahren folgende Schritte umfasst:
a) Emulgieren oder Dispergieren einer hydrophoben Flüssigkeit, eines in einer hydrophoben Flüssigkeit suspendierten Feststoffs oder eines Feststoffs in eine Lösung von mindestens einem Hochmolekulargewichts-Protein;
b) Unterwerfen der in Schritt a) erhaltenen Emulsion oder Dispersion einer Verwässerung und/oder einer pH-Einstellung zum Erhalten einer Koazervierung;
c) Abkühlen der in Schritt b) erzeugten Koazervierung, um eine Wand-Bildung von Mikrokapseln bereitzustellen;
d) bei einer Temperatur von zwischen 15 und 30°C, Hinzufügen eines Härtungsmittels zur Koazervierung, um die gebildete Wand zu vernetzen; und
e) Schütteln der Mischung während mindestens 48 Stunden, um die Vernetzungsreaktion abzuschließen;
**dadurch gekennzeichnet, dass** das in Schritt d) verwendete Härtungsmittel mindestens einen Pflanzenextrakt, umfassend substituierte und unsubstituierte Phenolverbindungen, umfasst.

2. Verfahren nach Anspruch 1, **dadurch gekennzeichnet, dass** das Härtungsmittel 0,5 bis 2,5 Gew.-% im Verhältnis zum Gewicht des Proteins, auf trockener Basis, darstellt.

3. Verfahren nach Anspruch 1, **dadurch gekennzeichnet, dass** das Härtungsmittel mindestens einen Pflanzenextrakt, ausgewählt aus der Gruppe bestehend aus botanischen Extrakten, Kräuterextrakten und Holzextrakten, umfasst.

4. Verfahren nach Anspruch 3, **dadurch gekennzeichnet, dass** das Härtungsmittel einen konzentrierten Teeextrakt enthällt.

5. Verfahren nach Anspruch 3, **dadurch gekennzeichnet, dass** das Härtungsmittel Holzessig enthält.

6. Verfahren nach Anspruch 1, **dadurch gekennzeichnet, dass** das hydrophobe Kernmaterial ein duftender oder aromatischer Inhaltsstoff oder Zusammensetzung ist.

7. Verfahren nach Anspruch 1, **dadurch gekennzeichnet, dass** die Proteinlösung ferner ein anionisches Polymer umfasst.

8. Verfahren nach Anspruch 7, **dadurch gekennzeichnet, dass** das anionische Polymer ausgewählt ist aus der Gruppe bestehend aus Gummiarabikum, Natriumalginat, Agar, Carrageenan, Carboxymethylcellulose, Natriumpolyacrylat oder Polyphosphorsäure.

9. Verfahren nach Anspruch 1, **dadurch gekennzeichnet, dass** das Protein aus Gelatin besteht.

10. Mikrokapseln, die durch ein Verfahren nach Anspruch 1 erhalten wurden.

11. Mikrokapseln umfassend gelierte Wände eines Hochmolekulargewichts-Proteins, die wenigstens teilweise die Kerne des hydrophoben Materials umgeben, wobei die gelierten Wände durch ein Härtungsmittel vernetzt sind, das mindestens einen Pflanzenextrakt, der substituierte oder unsubstituierte Phenolverbindungen umfasst, enthält.

12. Mikrokapseln nach Anspruch 11, **dadurch gekennzeichnet, dass** das Protein Gelatin oder Albumin ist und der Pflanzenextrakt ausgewählt ist aus der Gruppe bestehend aus botanischen Extrakten, Kräuterextrakten und Holzextrakten.

13. Verfahren zum Insolubilisieren der Proteine in Wasser, was das Hinzufügen von mindestens einem Pflanzenextrakt, umfassend substituierte oder unsubstituierte Phenolverbindungen, zu einer auf einem Protein basierten Koazervierung umfasst, um eine Mischung zu bilden, wobei der Extrakt in einer Menge anwesend ist, die ausreichend ist, um mindestens einen Teil des Proteins der Koazervierung zu härten, um es unlöslich zu machen.

14. Verfahren zum Verleihen, Verbessern oder Modifizieren der organoleptischen Eigenschaften einer duftenden oder aromatischen Zusammensetzung, was das Hinzufügen von Mikrokapseln nach Anspruch 10 zu der Verbindung umfasst.

15. Duftende Zusammensetzung oder duftender Artikel umfassend, zusammen mit in der Parfümerie gebräuchlichen duftenden zusätzlichen Inhaltsstoffen, Lösungsmitteln oder Hilfsstoffen, Mikrokapseln nach Anspruch 10.

16. Duftender Artikel nach Anspruch 15, in Form eines Haushaltsreinigers, eines vorbefeuchteten Wischtuchs oder eines Körperpflegeartikels.

17. Duftende Zusammensetzung oder duftendes Produkt umfassend, zusammen mit in der Aromaindustrie gebräuchlichen aromatischen zusätzlichen Inhaltsstoffen oder Hilfsstoffen, Mikrokapseln nach Anspruch 10.

18. Aromatisches Produkt nach Anspruch 17, in Form einer Backware, einer Tabakware, eines gebratenen Produkts oder einer Konserve.

## Revendications

1. Procédé pour la préparation de microcapsules comprenant un noyau de matériau hydrophobe, lequel procédé comprend les étapes consistant à:
a) émulsifier ou disperser un liquide hydrophobe, un solide en suspension dans un liquide hydrophobe ou un solide dans une solution d'au moins une protéine de poids moléculaire élevé;
b) soumettre l'émulsion ou la dispersion obtenue dans l'étape a) à une dilution aqueuse et/ou un ajustement de pH pour obtenir une coacervation;
c) refroidir le coacervat formé à l'étape b) pour permettre la formation de la paroi des microcapsules;
d) ajouter, à une température entre 15 et 30°C, un agent durcisseur au coacervat pour réticuler le mur formé; et
e) agiter le mélange durant au moins 48 h pour achever la réaction de réticulation;
**caractérisé en ce que** l'agent durcisseur utilisé à l'étape d) contient au moins un extrait de plante comprenant des composés phénoliques substitués ou non substitués.

2. Procédé selon la revendication 1, **caractérisé en ce que** l'agent durcisseur représente de 0,5 à 2,5 % en poids par rapport au poids de la protéine, sur une base sèche.

3. Procédé selon la revendication 1, **caractérisé en ce que** l'agent durcisseur contient au moins un extrait de plante sélectionné dans le groupe constitué d'extraits botaniques, d'extraits végétaux et d'extraits de bois.

4. Procédé selon la revendication 3, **caractérisé en ce que** l'agent durcisseur contient un extrait de thé concentré.

5. Procédé selon la revendication 3, **caractérisé en ce que** l'agent durcisseur contient de l'acide pyroligneux.

6. Procédé selon la revendication 1, **caractérisé en ce que** le noyau de matériau hydrophobe est un ingrédient ou une composition parfumante ou aromatisante.

7. Procédé selon la revendication 1, **caractérisé en ce que** la solution protéinique comprend en outre un polymère anionique.

8. Procédé selon la revendication 7, **caractérisé en ce que** le polymère anionique est sélectionné dans le groupe constitué de gomme arabique, alginate de sodium, agar, carraghénane, carboxyméthylcellulose, polyacrylate de sodium ou acide polyphosphorique.

9. Procédé selon la revendication 1, **caractérisé en ce que** la protéine est la gélatine.

10. Microcapsules obtenues par un procédé selon la revendication 1.

11. Microcapsules comprenant des murs gélifiés d'une protéine de haut poids moléculaire qui entourent au moins partiellement des noyaux de matériau hydrophobe, dans lesquelles les murs gélifiés sont réticulés au moyen d'un agent durcisseur contenant au moins un extrait de plante comprenant des composés phénoliques substitués ou non substitués.

12. Microcapsules selon la revendication 11, **caractérisées en ce que** la protéine est la gélatine ou l'albumine et l'extrait de plante est sélectionné dans le groupe constitué d'extraits botaniques, d'extraits végétaux et d'extraits de bois.

13. Méthode d'insolubilisation de protéines dans de l'eau, qui comprend l'ajout d'au moins un extrait de plante comprenant des composés phénoliques substitués ou non substitués à un coacervat à base de protéine pour former un mélange dans lequel l'extrait est présent dans une quantité suffisante pour durcir au moins une portion de la protéine du coacervat pour la rendre insoluble.

14. Méthode pour conférer, améliorer ou modifier les propriétés organoleptiques d'une composition parfumante ou aromatisante, comprenant l'ajout à la composition de microcapsules selon la revendication 10.

15. Composition parfumante ou article parfumé comprenant, outre les co-ingrédients parfumants, solvants ou adjuvants d'usage courant en parfumerie, des microcapsules selon la revendication 10.

16. Article parfumé selon la revendication 15, sous la forme d'un nettoyant ménager, d'une lingette préimprégnée ou d'un produit de soins corporels.

17. Composition aromatisante ou produit aromatisé comprenant, outre les co-ingrédients aromatisants, ou adjuvants d'usage courant dans l'industrie des arômes, des microcapsules selon la revendication 10.

18. Produit aromatisé selon la revendication 17, sous la forme d'un produit de boulangerie, de tabac, de friture ou de conserve.
